# Europäisches Patentamt
# European Patent Office
# Office européen des brevets

(19)

(11) Veröffentlichungsnummer: **0 219 073**
**B1**

(12) **EUROPÄISCHE PATENTSCHRIFT**

(45) Veröffentlichungstag der Patentschrift:
07.03.90

(51) Int. Cl.⁴: **A61K 37/66, A61K 47/00**

(21) Anmeldenummer: **86114047.3**

(22) Anmeldetag: **10.10.86**

(54) **Stabilisierung von Interferonen.**

(30) Priorität: **15.10.85 CH 4433/85**

(43) Veröffentlichungstag der Anmeldung:
**22.04.87 Patentblatt 87/17**

(45) Bekanntmachung des Hinweises auf die Patenterteilung:
**07.03.90 Patentblatt 90/10**

(84) Benannte Vertragsstaaten:
**AT BE CH DE FR GB IT LI LU NL SE**

(56) Entgegenhaltungen:
EXCERPTA MEDICA, vol. 80, no. 14733; P.JAMESON et al.: "Thermal stability of freeze-dried mammalian interferons. Analysis of freeze-drying conditions and accelerated storage tests for murine interferon"

(73) Patentinhaber: **F. HOFFMANN-LA ROCHE AG,
Postfach 3255, CH-4002 Basel(CH)**

(72) Erfinder: **Galiati, Harald, Dr., Ingelsteinweg 13,
CH-4143 Dornach(CH)**
Erfinder: **Pracht, Inge, Rössligasse 41,
CH-4125 Riehen(CH)**

(74) Vertreter: **Lederer, Franz, Dr. et al, Van der Werth,
Lederer & Riederer Patentanwälte
Lucile-Grahn-Strasse 22, D-8000 München 80(DE)**

## Beschreibung

Die vorliegende Erfindung betrifft Interferon-Zusammensetzungen mit verbesserter Stabilität der biologischen und immunologischen Interferon-Aktivität sowie Verfahren zu deren Herstellung.

Unter Interferonen versteht man eine Gruppe körpereigener Proteine mit antiviraler und immunregulatorischer Wirkung. Der antivirale Effekt wird dabei nicht durch eine direkte Beeinflussung der Viren selbst erzielt sondern durch eine Wirkung auf deren Zielzellen im Sinne eines Schutzes gegen die Virusinfektion. Neben der antiviralen Aktivität können die Interferone objektivierbare Effekte auf Krebsgeschwülste ausüben, die sie für den Einsatz in der Krebstherapie geeignet machen und sie beeinflussen das körpereigene Immunsystem, indem sie z.B. Makrophagen und NK-Zellen aktivieren und die Expression verschiedener immunologisch bedeutsamer Bestandteile der Zellmembran verstärken.

Interferone (IFN-$\alpha$, -$\beta$ und -$\gamma$) können heute dank rekombinanter DNA-Technologie auf mikrobiellem Wege in Mengen hergestellt werden, die früher trotz grösster Anstrengungen durch Isolierung aus natürlichem Material (Leukocyten, Fibroblasten, Lymphocyten) und Reinigung nicht zur Verfügung gestellt werden konnten.

Erst diese neue Technologie hat daher einen Weg für die intensive klinische Erprobung und allfällige breite therapeutische Anwendung der Interferone geöffnet und macht eine hinreichende Versorgung der für eine Behandlung infrage kommenden Personen mit den Wirksubstanzen möglich.

Unter den Human-Interferonen ist das IFN-$\gamma$ das instabilste Protein, dessen biologische Aktivität durch Lagerung, Einfrieren oder Lyophilisation schnell verloren geht. Diese Instabilität erschwert in erheblichem Masse die Herstellung, Reinigung, Lagerung und therapeutische Anwendung des IFN-$\gamma$. Die Stabilisierung seiner biologischen Aktivität spielt daher eine besonders wichtige Rolle. Im Vergleich zum IFN-$\gamma$ sind die Human-Interferone $\alpha$ und $\beta$ relativ stabile Proteine und können ohne nennenswerten Verlust an biologischer Aktivität gereinigt, gelagert und zur Therapie verwendet werden.

Zur Stabilisierung von Interferonen, insbesondere IFN-$\gamma$ wurden bisher verschiedene Hilfsstoffe herangezogen. So wurde die Stabilisierung von Interferonen mittels Propylenglycol im U.S. Patent Nr. 4 483 849 beschrieben. Es stellte sich jedoch heraus, dass Propylenglycol zur Stabilisierung von rekombinantem IFN-$\gamma$ nicht effizient eingesetzt werden kann. Auch die Stabilisierung von IFN-$\gamma$ mittels Rinderserumalbumin (Rinderknecht et al., J. Biol. Chem. 259, 6790-6797 [1984]) wurde geprüft und für nicht effizient befunden. Die Stabilisierung von IFN-$\gamma$ mittels 0,1% BSA und 0,5% Gelatine (Devos et al., J. Interferon Res. 4, 461-468 [1984]) ist ebenfalls bekannt. Um einen Aktivitätsverlust zu vermeiden, müssen derartige Präparate aber immer noch bei -70°C aufbewahrt werden.

Aus Cryobiology 16, 301–314 (1979) sind Mäuse-Interferon enthaltende Zusammensetzungen bekannt, die ein Phosphatpuffersystem mit einem pH-Wert von 7 und einer Phosphatkonzentration von 0,1 M und 0,5% Rinderserumalbumin enthalten.

Erfindungsgemäss wurde nun gefunden, dass Interferone in Gegenwart einer hohen Konzentration an Phosphat und/oder Polyphosphat eine ausgezeichnete Stabilität aufweisen, so dass die Interferone in Lösungen ohne nennenswerten Verlust an biologischer und immunologischer Aktivität bei 2-8°C gelagert werden können.

Die vorliegende Erfindung betrifft daher Zusammensetzungen, enthaltend Interferon, ein Phosphatpuffersystem und/oder Polyphosphat, sowie ein Verfahren zu deren Herstellung, das darin besteht, dass man eine Interferon-Lösung mit Phosphat und/oder Polyphosphat versetzt.

Die vorliegende Erfindung betrifft ausserdem pharmazeutische Präparate auf der Basis der erfindungsgemässen Zusammensetzung, sowie ein Verfahren zur Herstellung derartiger pharmazeutischer Präparate, welches dadurch gekennzeichnet ist, dass man eine erfindungsgemässe Zusammensetzung und, erwünschtenfalls, einen oder mehrere andere therapeutisch wertvolle Stoffe mit einem pharmazeutisch annehmbaren Träger zusammenmischt und das anfallende Gemisch in eine geeignete Dosierungsform gebracht wird.

Die erfindungsgemässe Stabilisierung kann auf alle Interferone oder Polypeptide mit Interferon-ähnlichen biologischen Eigenschaften sowie auf Gemische, die ein Interferon oder ein solches Polypeptid enthalten, angewendet werden. Vorzugsweise wird sie bei Human-Interferon angewendet werden. Das Human-Interferon kann ein natürliches oder rekombinantes Leukozyten-Interferon (IFN-$\alpha$), Fibroblasten-Interferon (IFN-$\beta$) oder Immun-Interferon (IFN-$\gamma$) sein. Auch sogennante hybride Interferone, bei denen Fragmente von zwei oder mehreren nativen Interferon-Species verknüpft werden, können erfindungsgemäss stabilisiert werden. Ein besonders bevorzugtes Human-Interferon im Zusammenhang mit der vorliegenden Erfindung ist IFN-$\gamma$, vorzugsweise rekombinantes IFN-$\gamma$, speziell IFN-$\gamma$ D0 oder IFN-$\gamma$ D3. Die Bezeichnungen D0 und D3 dienen zur Unterscheidung der letztgenannten Interferone, wobei mit D0 dasjenige rIFN-$\gamma$ bezeichnet wird, dessen N-terminale Aminosäuresequenz mit Cys-Tyr-Cys bzw. Met-Cys-Tyr-Cys beginnt, während unter D3 ein um Cys-Tyr-Cys verkürztes rIFN-$\gamma$ zu verstehen ist, dessen N-terminale Aminosäuresequenz mit Gln bzw. Met-Gln beginnt.

Der Gehalt der erfindungsgemässen Zusammensetzungen an Interferon ist nicht kritisch. Der Konzentrationsbereich erstreckt sich über mehrere Zehnerpotenzen und wird nach oben im wesentlichen nur durch die Löslichkeit des betreffenden Interferons beschränkt. Für Human-IFN-$\gamma$ kommen biespielsweise Konzentrationen biz zu $10^8$ Einheiten (E)/ml in Frage, wobei ein bevorzugter Bereich $4 \cdot 10^3$ - etwa $10^6$ E/ml beträgt.

Die erfindungsgemässen Zusammensetzungen können zur Herstellung eines pharmazeutischen Präparates zur Therapie bzw. Prophylaxe von viralen Infektionen und immunregulatorischer Anomalien, insbesondere Neoplasmen verwendet werden.

Das Phosphatpuffersystem der vorliegenden Erfindung ist so eingerichtet, dass es ein pH von etwa 6,0-9,0, vorzugsweise von 7,5 aufrechterhält und eine Phosphatkonzentration von 500-1000 mMol/l, vorzugsweise 1000 mMol/l, aufweist. Als Phosphatquelle kommen Kaliumphosphate und Natriumphosphate, vorzugsweise Dinatriumhydrogenphosphat und Mononatriumhydrogenphosphat, in Frage. Ein besonders bevorzugtes Phosphatpuffersystem der vorliegenden Erfindung ist so eingerichtet, dass es ein pH von etwa 7,5 gewährleistet und eine Phosphatkonzentration von 500-1000 mMol/l, vorzugsweise 1000 mMol/l, sowie 25-100 g/l, vorzugsweise 50 g/l, Polyphosphat, speziell Kaliumpolyphosphat, enthält.

Die Polyphosphat-Lösungen vom pH 7,5 der vorliegenden Erfindung, werden in einer Konzentration von 25-100 g/l, vorzugsweise 50 g/l Polyphosphat, speziell Kaliumpolyphosphat eingesetzt.

In den folgenden Beispielen werden Einzelheiten der Erfindung beschrieben.

Die in den Beispielen verwendeten Chemikalien waren p.a. oder von höchster Reinheit.

Alle erfindungsgemäss stabilisierbaren Interferone, insbesondere das in den Beispielen verwendete rekombinante IFN-γ D3 können in reiner Form nach bekannten, in der Literatur beschriebenen Verfahren oder nach für den Fachmann naheliegenden Verfahren erhalten werden.

Zur Bestimmung der immunologischen Aktivität des rekombinanten IFN-γ D3 wurde ein Enzym-immunologischer Test (IFN-γ-EIA) eingesetzt, der dem Enzym-immunologischen Test von H. Gallati zur Bestimmung des Human-Leukozyten-Interferons, der im J. Clin. Chem. Clin. Biochem. 20, 907-914 (1982) beschrieben ist, entspricht.

Für die Stabilitätsprüfung des IFN-γ wurden Lösungen mit 4000-10000 E/ml IFN-γ und den zu untersuchenden Zusätzen in Glasampullen bei +37°C inkubiert und nach gewissen Zeitintervallen, die in der inkubierten IFN-γ-Lösung noch vorhandene immunologische Aktivität des IFN-γ mit dem IFN-γ-EIA bestimmt.

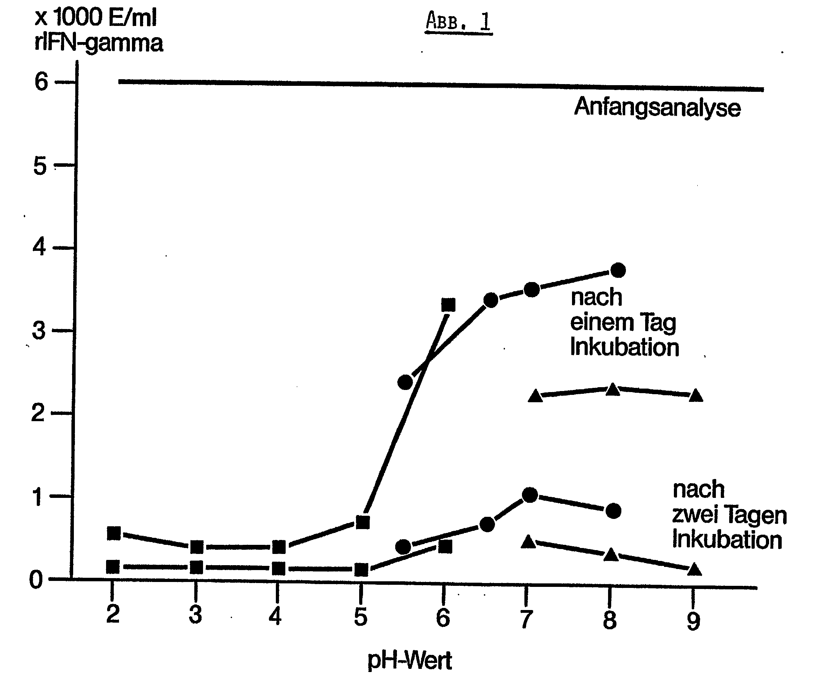

Abb. 1 zeigt die Stabilität der immunologischen Aktivität des IFN-γ in Abhängigkeit vom Puffersystem und pH-Wert. 6000 E/ml IFN-γ wurden in je 0,1 Mol/l Natriumacetat vom pH 2,0-6,0

(■————■) ,

Natriumphosphat vom pH 5,5-8,0 (●-●) und in TRIS/Acetat vom pH 7,2-9,0

(▲————————▲)

aufgenommen und während 2 Tagen in verschlossenen Glasampullen bei 37°C inkubiert. Die immunologische Aktivität des IFN-γ wurde vor der Inkubation sowie einen und 2 Tage nach Inkubation mit dem IFN-γ-EIA bestimmt.

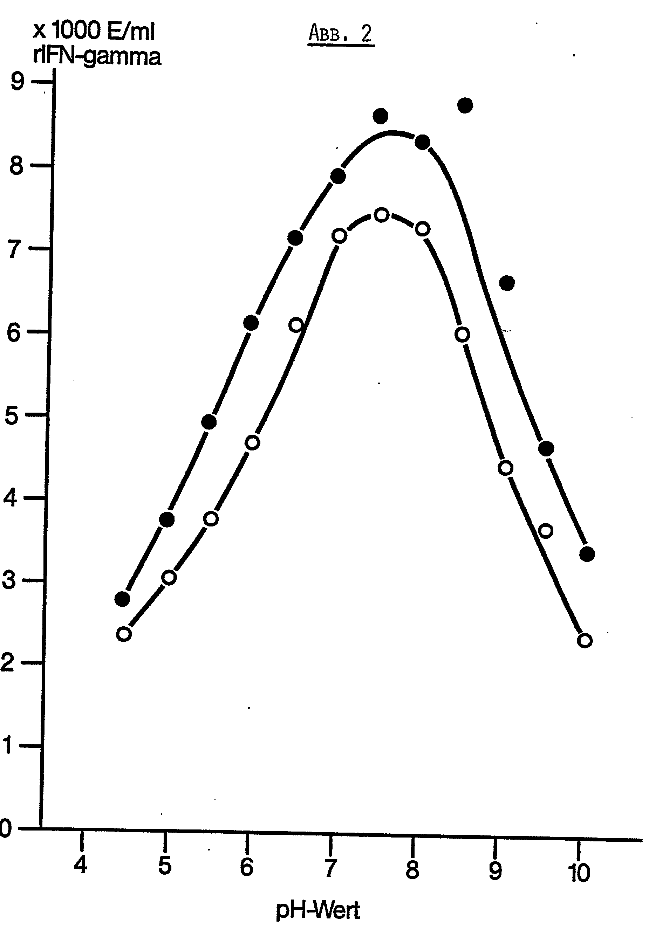

Abb. 2 zeigt die Stabilität der immunologischen Aktivität des IFN-γ in 500 mMole/l Natriumphosphat in Abhängigkeit vom pH-Wert. 10000 E/ml

IFN-γ wurden in 500 mMol/l Natriumphosphatpuffer vom pH 4,5-10 aufgenommen und in Glasampullen während 7 Tagen bei 37°C inkubiert. Nach 3 Tagen (●———●) und nach Tagen Inkubation (o———o) wurde die immunologische Aktivität mit dem IFN-γ-EIA bestimmt.

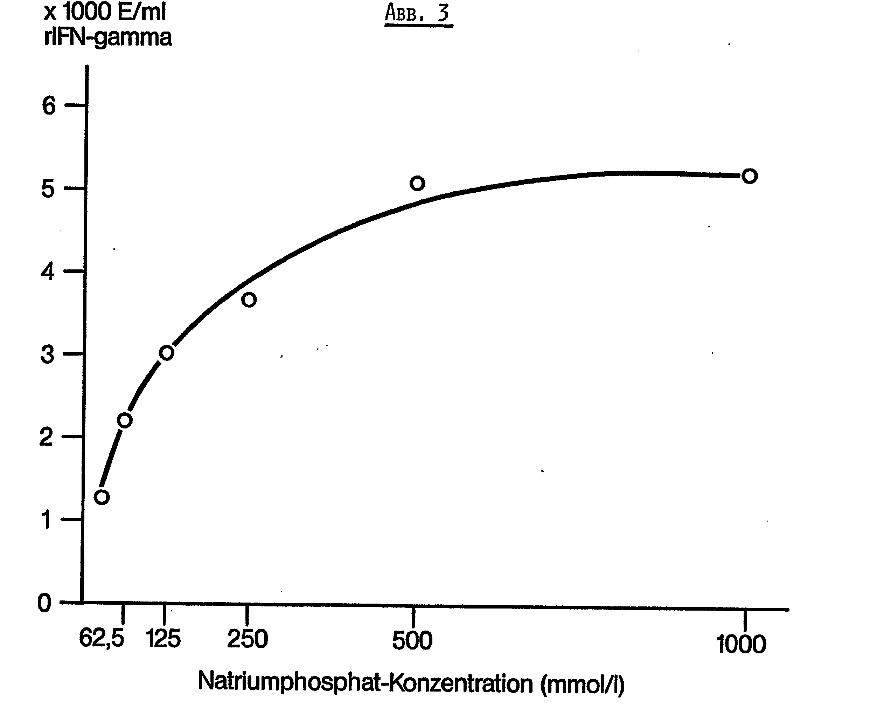

Abb.3 zeigt die Stabilität der immunologischen Aktivität des IFN-γ beim pH-Wert 7,5 in Abhängigkeit von der Natriumphosphatkonzentration. 6000 E/ml IFN-γ wurden in Lösungen aufgenommen, die mit unterschiedlicher Natriumphosphatkonzentration gepuffert und auf den pH-Wert 7,5 eingestellt wurden. Die IFN-γ-Lösungen wurden in Glasampullen während 7 Tagen bei 37°C inkubiert. Nach dieser Inkubation wurde die immunologische Aktivität in den IFN-γ-Lösungen mit dem IFN-γ-EIA bestimmt.

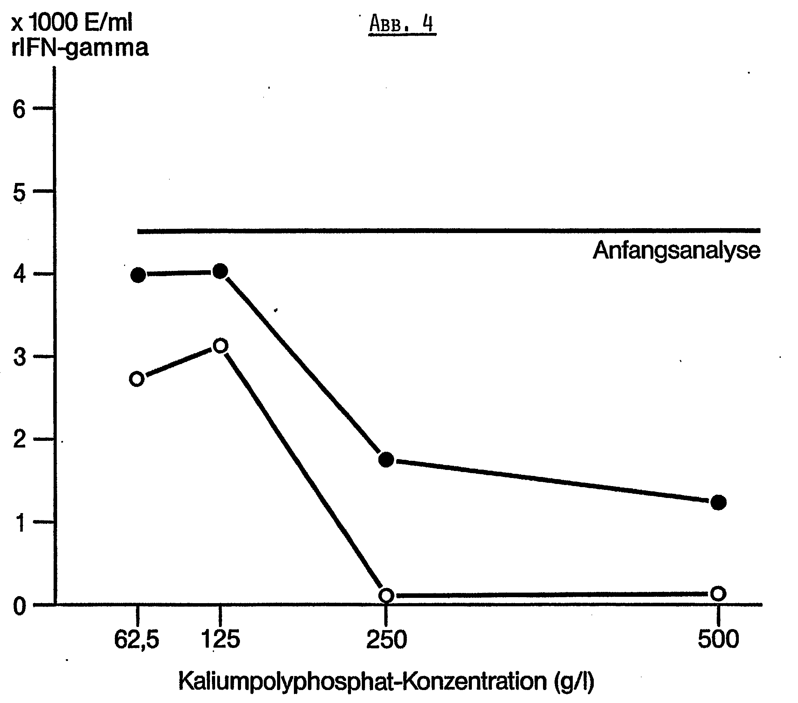

Abb. 4 zeigt die Stabilität der immunologischen Aktivität des IFN-γ in Abhängigkeit von der Polyphosphat-Konzentration in der Lösung. 4500 E/ml IFN-γ wurden in Lösungen aufgenommen, die verschiedene Konzentrationen von Kaliumpolyphosphat, pH 7,5, enthielten. Diese IFN-γ-Lösungen wurden in Glasampullen während 16 Tagen bei 37°C inkubiert. Nach 3 Tagen (●———●) und nach 16 Tagen Inkubation (o———o) wurde die immunologische Aktivität des IFN-γ in den betreffenden Lösungen mit dem IFN-γ-EIA bestimmt.

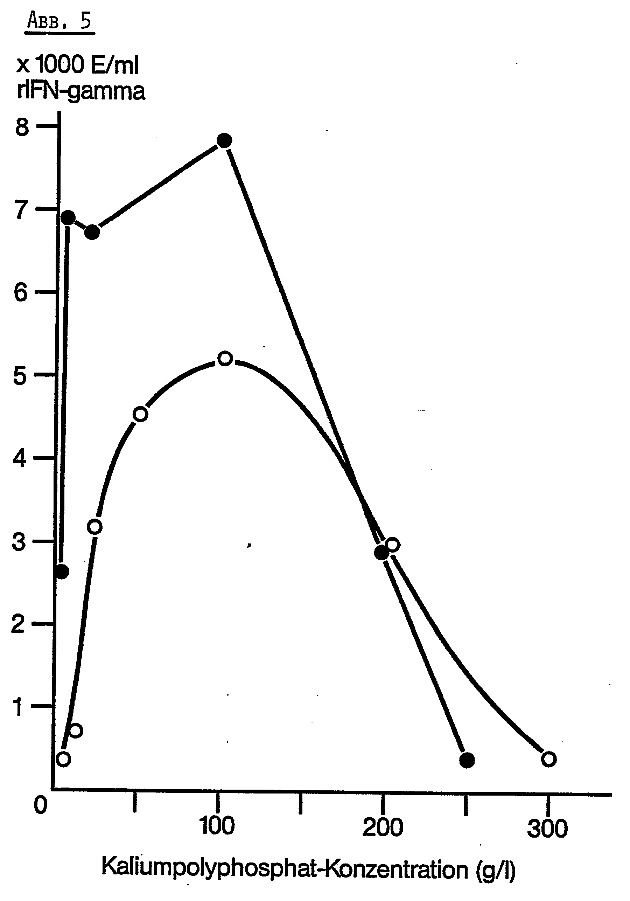

Abb. 5 zeigt die Stabilität der immunologischen Aktivität des IFN-γ in Abhängigkeit von der Polyphosphatkonzentration bei Anwesenheit und bei Abwesenheit von Natriumphosphat. 8000 E/ml IFN-γ wurden in Lösungen aufgenommen, die unterschiedliche Konzentrationen an Kaliumpolyphosphat vom pH 7,5 enthielten (o———o). Zusätzlich wurde ein Teil der Lösungen mit 1000 mMol/l Natriumphosphat vom pH 7,5 gepuffert (●———●). Die in dieser Abbildung aufgetragenen IFN-γ-Aktivitäten wurden nach einer 20-tägigen Inkubation bei 37°C mit dem IFN-γ-EIA gemessen.

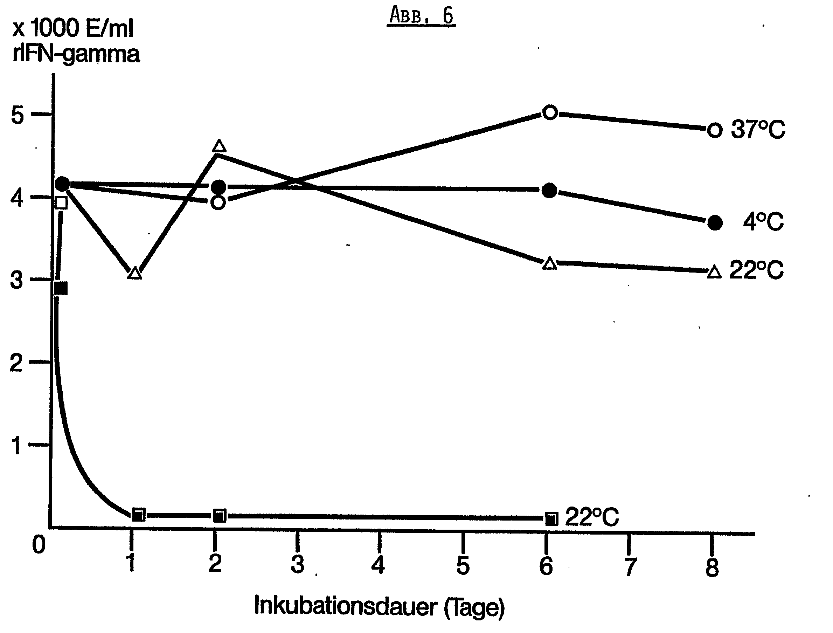

Abb. 6 zeigt vergleichende Stabilitätstests mit IFN-γ in verschiedenen Lösungsmitteln und bei verschiedenen Temperaturen. Je 4000 E/ml IFN-γ wurden aufgenommen in

a) 20 mMol/l Natriumphosphat mit 9 g/l NaCl und 0,25 g/l Thimerosal (Natrium-Ethylmercurithiosalicylat, Fa. Fluka), pH 7,5

(□————□) ,

b) 20 mMol/l Natriumphosphat mit 9 g/l NaCl, 5 g/l BSA und 0,25 g/l "Thimerosal", pH 7,5

(■ ————■) ,

und
c) 1000 mMol/l Natriumphosphat, 50 g/l Kaliumpolyphosphat und 0,25 g/l "Thimerosal", pH 7,5

(Δ————Δ,

o———o, ●———●).

Diese IFN-γ-Lösungen wurden in Glasampullen während 8 Tagen bei 2-8°C, 22°C und +37°C inkubiert. Nach bestimmten Zeitintervallen wurde die immunologische Aktivität des IFN-γ mit dem IFN-γ-EIA bestimmt.

Beispiel 1

In einer ersten Versuchsserie wurde die IFN-γ-Stabili- tät in verschiedenen Puffersystemen und bei verschiedenen pH-Werten untersucht. 6000 E/ml IFN-γ wurden in je 0,1 Mol/l Natriumacetat vom pH 2,0-6,0, Natriumphosphat vom pH 5,5-8,0 und in TRIS/Acetat vom pH 7,2-9,0 aufgenommen und während 2 Tagen in verschlossenen Glasampullen bei 37°C inkubiert. Die immunologische Aktivität des IFN-γ wurde vor der Inkubation sowie einen und 2 Tage nach Inkubation mit dem IFN-γ-EIA bestimmt. Die in Abb. 1 dargestellten Resultate zeigen generell eine schlechte Stabilität der immunologischen Aktivität des IFN-γ in 0,1 Mol/l der untersuchten Puffersysteme. Die besten Ergebnisse werden mit dem Phosphatpuffersystem erreicht.

Beispiel 2

In einer weiteren Serie wurde die IFN-γ-Stabilität in 500 mMol/l Natriumphosphat in Abhängigkeit zum pH-Wert (4,5-10) untersucht. Jeweils 10'000 E/ml IFN-γ wurden in 500 mMol/l Natriumphosphatpuffer vom pH 4,5-10 aufgenommen und in Glasampullen während 7 Tagen bei 37°C inkubiert. Nach 3 Tagen und nach 7 Tagen Inkubation wurde die immunologische Aktivität mit dem IFN-γ-EIA bestimmt. Es wurde festgestellt, dass einerseits mit der erhöhten Phosphatkonzentration generell eine wesentlich bessere Stabilität erreicht werden kann und dass andererseits innerhalb des untersuchten pH-Bereichs von 4,5-10 das IFN-γ beim pH 7,5 am stabilsten ist (Abb. 2). So konnten nach einer 7-tägigen Inkubation bei 37°C in einer mit 500 mMol/l Natriumphosphat-gepufferten Lösung vom pH 7,5 noch 70% der ursprünglichen immunologischen Aktivität des eingesetzten IFN-γ gefunden werden.

Beispiel 3

Auf Grund der überraschenden Resultate aus Beispiel 2 wurde der Einfluss der Natriumphosphatkonzentration beim pH 7,5 auf die IFN-γ-Stabilität bei einer Inkubationstemperatur von +37°C untersucht. 6000 E/ml IFN-γ wurden in Lösungen aufgenommen, die mit unterschiedlicher Natriumphosphatkonzentration gepuffert und auf den pH-Wert 7,5 eingestellt waren. Die IFN-γ-Lösungen wurden in Glasampullen während 7 Tagen bei 37°C inkubiert. Nach dieser Inkubationsdauer wurde die immunologische Aktivität in den IFN-γ-Lösungen mit dem IFN-γ-EIA bestimmt. Die Resultate, die in der Abb. 3 zusammengestellt sind, bestätigen die in Abb. 1 und 2 dargestellten Befunde und zeigen mit zunehmender Phosphatkonzentration eine wesentliche Verbesserung der IFN-γ-Stabilität.

Beispiel 4

Da die Natriumphosphatkonzentration aus Löslichkeitsgründen nicht wesentlich über 1000 mMol/l gesteigert werden kann (Auskristallisation bei 4°C), wurde in einer weiteren Versuchsserie der Einfluss von Polyphosphat auf die IFN-γ-Stabilität bei 37°C untersucht. 4500 E/ml IFN-γ wurde in Lösungen aufgenommen, die verschiedene Konzentrationen von Kaliumpolyphosphat, pH 7,5, enthielten. Diese IFN-γ-Lösungen wurden in Glasampullen während 16 Tagen bei 37°C inkubiert. Nach 3 und nach 16 Tagen wurde die immunologische Aktivität des IFN-γ in den betreffenden Lösungen mit dem IFN-γ-EIA bestimmt. Die Resultate (Abb. 4) zeigen einen ähnlich guten Effekt wie mit hohen Konzentration an Natriumphosphat.

Beispiel 5

Um den Einfluss einer Kombination von Kaliumpolyphosphat und von Natriumphosphat auf die Stabilität des IFN-γ abzuklären, wurden 8000 E/ml IFN-γ in Lösungen aufgenommen, die unterschiedliche Konzentrationen an Kaliumpolyphosphat vom pH 7,5 enthielten. Zusätzlich wurde ein Teil der Lösungen mit 1000 mMol/l Natriumphosphat vom pH 7,5 gepuffert. Die Resultate (Abb. 5) zeigen eine optimale Stabilisierung von IFN-γ mit einer Kombination von 1000 mMol/l Natriumphosphat vom pH 7,5 und 25-100 g/l, vorzugsweise 50 g/l Polyphosphat.

Beispiel 6

Abschliessend wurde die Stabilität des IFN-γ in diversen Lösungsmitteln untersucht. So wurden je 4000 E/ml IFN-γ aufgenommen in (a) 20 mMol/l Natriumphosphat mit 9 g/l NaCl und 0,25 g/l Thimerosal, pH 7,5 (b) 20 mMol/l Natriumphosphat mit 9 g/l NaCl, 5 g/l BSA und 0,25 g/l Thimerosal, pH 7,5, und (c) 1000 mMol/l Natriumphosphat, 50 g/l Kaliumpolyphosphat und 0,25 g/l Thimerosal, pH 7,5. Diese IFN-γ-Lösungen wurden in Glasampullen während 8 Tagen bei 2-8°C, 22°C und +37°C inkubiert. Nach bestimmten Zeitintervallen wurde die immunologische Aktivität des IFN-γ bestimmt. Wie aus Abb. 6 hervorgeht, zeigt der Vergleich der Lagerstabilitäten des IFN-γ in PBS, in PBS-BSA sowie in 1000 mMol/l Natriumphosphat/50 g/l Natriumpolyphosphat vom pH 7,5, dass die immunologische Aktivität des IFN-γ in PBS und in PBS-BSA bei einer Inkubationstemperatur von 22°C schon nach 1 Tag praktisch vollständig verloren geht, während sie in der Phosphat-Polyphosphatlösung nach 8 Tagen Inkubation bei 4°C, bei 22°C und bei 37°C fast vollständig erhalten bleibt.

**Patentansprüche für die Vertragsstaaten: BE, CH, DE, FR, GB, IT, LI, LU, NL, SE**

1. Zusammensetzung, enthaltend Interferon, ein Phosphatpuffersystem, das einen pH-Wert von 6.0-9.0, vorzugsweise von 7.5 gewährleistet und das eine Phosphatkonzentration von 500-1000 mMol/l, vorzugsweise 1000 mMol/l aufweist, und/oder Polyphosphat.

2. Zusammensetzung nach Anspruch 1, dadurch gekennzeichnet, daß das Phosphatpuffersystem Dinatriumhydrogenphosphat und Mononatriumhydrogenphosphat enthält.

3. Zusammensetzung nach Anspruch 1 oder 2, dadurch gekennzeichnet, daß sie 25–100 g/l Polyphosphat enthält.

4. Zusammensetzung nach Anspruch 1 oder 2, dadurch gekennzeichnet, daß sie 50 g/l Polyphosphat enthält.

5. Zusammensetzung nach Anspruch 1, dadurch gekennzeichnet, daß sie 25–100 g/l Polyphosphatlösung vom pH 7,5 enthält.

6. Zusammensetzung nach Anspruch 1, dadurch gekennzeichnet, daß sie 50 g/l Polyphosphatlösung vom pH 7,5 enthält.

7. Zusammensetzung nach Anspruch 5 oder 6, dadurch gekennzeichnet, daß sie Kaliumpolyphosphat enthält.

8. Zusammensetzung nach einem der Ansprüche 1–7, dadurch gekennzeichnet, daß das Interferon ein Human-Interferon ist.

9. Zusammensetzung nach Anspruch 8, dadurch gekennzeichnet, daß das Human-Interferon ein natürliches oder ein rekombinantes IFN-$\gamma$ ist.

10. Zusammensetzung nach Anspruch 9, dadurch gekennzeichnet, daß sie rIFN-$\gamma$ D0 enthält.

11. Zusammensetzung nach Anspruch 9, dadurch gekennzeichnet, daß sie rIFN-$\gamma$ D3 enthält.

12. Zusammensetzung enthaltend rekombinantes Human-IFN-$\gamma$ D3, 1000 mMol/l Natriumphosphatpuffer vom pH 7,5 und 50 g/l Kaliumpolyphosphat.

13. Zusammensetzung gemäß einem der Ansprüche 1–12, als pharmazeutischer Wirkstoff.

14. Zusammensetzung gemäß einem der Ansprüche 1–12, als antiviraler und immunoregulatorischer Wirkstoff.

15. Verfahren zur Herstellung einer Zusammensetzung nach einem der Ansprüche 1–12, dadurch gekennzeichnet, daß man eine Interferon-Lösung mit einem Phosphatpuffersystem gemäß Anspruch 1 und/oder Polyphosphat versetzt.

16. Pharmazeutische Präparate auf der Basis einer Zusammensetzung nach einem der Ansprüche 1–12.

17. Verwendung eines Phosphatpuffersystems nach Anspruch 1 und/oder von Polyphosphat zur Stabilisierung von Interferon.

18. Verwendung einer Zusammensetzung nach einem der Ansprüche 1–12 zur Herstellung eines pharmazeutischen Präparates zur Therapie bzw. Prophylaxe von Krankheiten.

19. Verwendung einer Zusammensetzung nach einem der Ansprüche 1–12 zur Herstellung eines pharmazeutischen Präparates zur Therapie bzw. Prophylaxe von viralen Infektionen.

20. Verwendung einer Zusammensetzung nach einem der Ansprüche 1–12 zur Herstellung eines pharmazeutischen Präparates zur Therapie bzw. Prophylaxe von immunregulatorischen Anomalien.

21. Verwendung einer Zusammensetzung nach einem der Ansprüche 1–12 zur Herstellung eines pharmazeutischen Präparates zur Therapie bzw. Prophylaxe von Neoplasmen.

**Patentansprüche für den Vertragsstaat: AT**

1. Verwendung eines Phosphatpuffersystems, das einen pH-Wert von 6,0-9,0, vorzugsweise von 7,5 gewährleistet und das eine Phosphatkonzentration von 500-1000 mMol/l, vorzugsweise 1000 mMol/l aufweist, oder von Polyphosphat zur Stabilisierung von Interferon.

2. Verwendung einer Zusammensetzung enthaltend Interferon, ein Phosphatpuffersystem, das einen pH-Wert von 6,0-9,0, vorzugsweise von 7,5 gewährleistet und das eine Phosphatkonzentration von 500-1000 mMol/l, vorzugsweise 1000 mMol/l aufweist, zur Herstellung eines pharmazeutischen Präparates zur Therapie bzw. Prophylaxe von Krankheiten.

3. Verwendung einer Zusammensetzung nach Anspruch 2 zur Herstellung eines pharmazeutischen Präparates zur Therapie bzw. Prophylaxe von viralen Infektionen.

4. Verwendung einer Zusammensetzung nach Anspruch 2 zur Herstellung eines pharmazeutischen Präparates zur Therapie bzw. Prophylaxe von immunregulatorischen Anomalien.

5. Verwendung einer Zusammensetzung nach Anspruch 2 zur Herstellung eines pharmazeutischen Präparates zur Therapie bzw. Prophylaxe von Neoplasmen.

6. Verwendung nach einem der Ansprüche 1-5, dadurch gekennzeichnet, daß das Phosphatpuffersystem Dinatriumhydrogenphosphat und Mononatriumhydrogenphosphat enthält.

7. Verwendung nach einem der Ansprüche 2-5, dadurch gekennzeichnet, daß die Zusammensetzung 25-100 g/l Polyphosphat enthält.

8. Verwendung nach Anspruch 7, dadurch gekennzeichnet, daß die Zusammensetzung 50 g/l Polyphosphat enthält.

9. Verwendung nach Anspruch 7, dadurch gekennzeichnet, daß die Zusammensetzung 25-100 g/l Polyphosphatlösung vom pH 7,5 enthält.

10. Verwendung nach Anspruch 9, dadurch gekennzeichnet, daß die Zusammensetzung 50 g/l Polyphosphatlösung vom pH 7,5 enthält.

11. Verwendung nach Anspruch 9 oder 10, dadurch gekennzeichnet, daß die Zusammensetzung Kaliumpolyphosphat enthält.

12. Verwendung nach einem der Ansprüche 1-11, dadurch gekennzeichnet, daß das Interferon ein Human-Interferon ist.

13. Verwendung nach Anspruch 12, dadurch gekennzeichnet, daß das Human-Interferon ein natürliches oder ein rekombinantes IFN-$\gamma$ ist.

14. Verwendung nach Anspruch 13, dadurch gekennzeichnet, daß das Interferon rIFN-$\gamma$ D0 enthält.

15. Verwendung nach Anspruch 13, dadurch gekennzeichnet, daß das Interferon rIFN-$\gamma$ D3 enthält.

16. Verwendung einer Zusammensetzung enthaltend rekombinantes Human-IFN-$\gamma$ D3, 1000 mMol/l Natriumphosphatpuffer vom pH 7,5 und 50 g/l Kaliumpolyphosphat nach einem der Ansprüche 2-5.

17. Verfahren zur Herstellung einer Zusammensetzung, enthaltend Interferon, ein Phosphatpuffersystem und/oder Polyphosphat, dadurch gekennzeichnet, daß man eine Interferon-Lösung mit Phosphat und/oder Polyphosphat versetzt.

**Claims for the Contracting States: BE, CH, DE, FR, GB, IT, LI, LU, NL, SE**

1. A composition containing interferon, a phosphate buffer system, which guarantees a pH value of 6.0–9.0, preferably of 7.5, and which has a phosphate concentration of 500–1000 mmol/l, preferably 1000 mmol/l, and/or polyphosphate.

2. A composition according to claim 1, characterized in that the phosphate buffer system contains disodium hydrogen phosphate and monosodium hydrogen phosphate.

3. A composition to claim 1 or 2, characterized in that it contains 25–100 g/l of polyphosphate.

4. A composition according to claim 1 or 2, characterized in that it contains 50 g/l of polyphosphate.

5. A composition according to claim 1, characterized in that it contains 25–100 g/l of polyphosphate solution of pH 7.5.

6. A composition according to claim 1, characterized in that it contains 50 g/l of polyphosphate solution of pH 7.5.

7. A composition according to claim 5 or 6, characterized in that it contains potassium polyphosphate.

8. A composition according to any one of claims 1–7, characterized in that the interferon is a human interferon.

9. A composition according to claim 8, characterized in that the human interferon is a natural or a recombinant IFN-γ.

10. A composition according to claim 9, characterized in that it contains rIFN-γ D0.

11. A composition according to claim 9, characterized in that it contains rIFN-γ D3.

12. A composition containing recombinant human IFN-γ D3, 1000 mmol/l of sodium phosphate buffer of pH 7.5 and 50 g/l of potassium polyphosphate.

13. A composition according to any one of claims 1–12 as a pharmaceutically active agent.

14. A composition according to any one of claims 1–12 as an antiviral and immunoregulatory agent.

15. A process for the manufacture of a composition according to any one of claims 1–12, characterized by treating an interferon solution with a phosphate buffer system in accordance with claim 1 and/or polyphosphate.

16. A pharmaceutical preparation based on a composition according to any one of claims 1–12.

17. The use of a phosphate buffer system according to claim 1 and/or of polyphosphate for the stabilization of interferon.

18. The use of a composition according to any one of claims 1–12 for the manufacture of a pharmaceutical preparation for the therapy or prophylaxis of diseases.

19. The use of a composition according to any one of claims 1–12 for the manufacture of a pharmaceutical preparation for the therapy or prophylaxis of viral infections.

20. The use of a composition according to any one of claims 1–12 for the manufacture of a pharmaceutical preparation for the therapy or prophylaxis of immunoregulatory anomalies.

21. The use of a composition according to any one of claims 1–12 for the manufacture of a pharmaceutical preparation for the therapy or prophylaxis of neoplasms.

**Claims for the Contracting State: AT**

1. The use of a phosphate buffer system, which guarantees a pH value of 6.0–9.0, preferably of 7.5, and which has a phosphate concentration of 500–1000 mmol/l, preferably 1000 mmol/l, or of polyphosphate for the stabilization of interferon.

2. The use of a composition containing interferon, a phosphate buffer system, which guarantees a pH value of 6.0–9.0, peferably of 7.5, and which has a phosphate concentration of 500–1000 mmol/l, preferably 1000 mmol/l, for the manufacture of a pharmaceutical preparation for the therapy or prophylaxis of diseases.

3. The use of a composition according to claim 2 for the manufacture of a pharmaceutical preparation for the therapy or prophylaxis of viral infections.

4. The use of a composition according to claim 2 for the manufacture of a pharmaceutical preparation for the therapy or prophylaxis of immunoregulatory anomalies.

5. The use of a composition according to claim 2 for the manufacture of a pharmaceutical preparation for the therapy or prophylaxis of neoplasms.

6. The use according to any one of claims 1–5, characterized in that the phosphate buffer system contains disodium hydrogen phosphate and monosodium hydrogen phosphate.

7. The use according to any one of claims 2–5, characterized in that the composition contains 25–100 g/l of polyphosphate.

8. The use according to claim 7, characterized in that the composition contains 50 g/l of polyphosphate.

9. The use according to claim 7, characterized in the composition contains 25–100 g/l of polyphosphate solution of pH 7.5.

10. The use according to claim 8, characterized in that the composition contains 50 g/l of polyphosphate solution of pH 7.5.

11. The use according to claim 9 or 10, characterized in that the composition contains potassium polyphosphate.

12. The use according to any one of claims 1–11, characterized in that the interferon is a human interferon.

13. The use according to claim 12, characterized in that the human interferon is a natural or a recombinant IFN-γ.

14. The use according to claim 13, characterized in that the interferon contains rIFN-γ D0.

15. The use according to claim 13, characterized in that the interferon contains rIFN-γ D3.

16. The use of a composition containing recombinant human IFN-γ D3, 1000 mmol/l of sodium phosphate buffer of pH 7.5 and 50 g/l of potassium polyphosphate according to any one of claims 2–5.

17. A process for the manufacture of a composition containing interferon, a phosphate buffer system and/or polyphosphate, characterized by treating an interferon solution with phosphate and/or polyphosphate.

**Revendications pour les Etats contractants: BE, CH, DE, FR, GB, IT, LI, LU, NL, SE**

1.- Composition contenant de l'interféron, un système tampon phosphaté assurant un pH de 6,0 à 9,0, de préférence de 7,5, et ayant une concentration en phosphate de 500 à 1.000 mmol/litre, de préférence de 1.000 mmol/litre, et/ou du polyphosphate.

2.- Composition selon la revendication 1, caractérisée en ce que le système tampon phosphaté contient du phosphate disodique et du phosphate monosodique.

3.- Composition selon la revendication 1 ou 2, caractérisée en ce qu'elle contient de 25 à 100 g/l de polyphosphate.

4.- Composition selon la revendication 1 ou 2, caractérisée en ce qu'elle contient 50 g/l de polyphosphate.

5.- Composition selon la revendication 1, caractérisée en ce qu'elle contient de 25 à 100 g/l d'une solution de polyphosphate à pH 7,5.

6.- Composition selon la revendication 1, caractérisée en ce qu'elle contient 50 g/l d'une solution de polyphosphate à pH 7,5.

7.- Composition selon la revendication 5 ou 6, caractérisée en ce qu'elle contient du polyphosphate de potassium.

8.- Composition selon l'une des revendications 1 à 7, caractérisée en ce que l'interféron est un interféron humain.

9.- Composition selon la revendication 8, caractérisée en ce que l'interféron humain est un IFN-γ naturel ou recombinant.

10.- Composition selon la revendication 9, caractérisée en ce qu'elle contient du rIFN-γ D0.

11.- Composition selon la revendication 9, caractérisée en ce qu'elle contient du rIFN-γ D3.

12.- Composition contenant de l'IFN-γ D3 humain recombinant, 1.000 mmol/l de tampon au phosphate de sodium à pH 7,5 et 50 g/l de polyphosphate de potassium.

13.- Composition selon l'une des revendications 1 à 12 en tant que substance active pharmaceutique.

14.- Composition selon l'une des revendications 1 à 12 en tant que substance active antivirale et immunorégulatrice.

15.- Procédé de préparation d'une composition selon l'une des revendications 1 à 12, caractérisé en ce que l'on ajoute un système tampon phosphaté selon la revendication 1 et/ou du polyphosphate à une solution d'interféron.

16.- Compositions pharmaceutiques à base d'une composition selon l'une des revendications 1 à 12.

17.- Utilisation d'un système tampon phosphaté selon la revendication 1 et/ou d'un polyphosphate pour la stabilisation de l'interféron.

18.- Utilisation d'une composition selon l'une des revendications 1 à 12 pour la préparation d'une composition pharmaceutique pour la thérapie ou la prophylaxie de maladies.

19.- Utilisation d'une composition selon l'une des revendications 1 à 12 pour la préparation d'une composition pharmaceutique pour la thérapie ou la prophylaxie des infections virales.

20.- Utilisation d'une composition selon l'une des revendications 1 à 12 pour la préparation d'une composition pharmaceutique pour la thérapie ou la prophylaxie des anomalies immunorégulatrices.

21.- Utilisation d'une composition selon l'une des revendications 1 à 12 pour la préparation d'une composition pharmaceutique pour la thérapie ou la prophylaxie des néoplasmes.

**Revendications pour l'Etat contractant: AT**

1.- Utilisation d'un système tampon phosphaté assurant un pH de 6,0 à 9,0, de préférence de 7,5, et ayant une concentration en phosphate de 500 à 1.000 mmol/l, de préférence de 1.000 mmol/l, ou d'un polyphosphate pour la stabilisation de l'interféron.

2.- Utilisation d'une composition contenant de l'interféron, un système tampon phosphaté assurant un pH de 6,0 à 9,0, de préférence de 7,5, et ayant une concentration en phosphate de 500 à 1.000 mmol/l, de préférence de 1.000 mmol/l, pour la préparation d'une composition pharmaceutique pour la thérapie ou la prophylaxie de maladies.

3.- Utilisation d'une composition selon la revendication 2 pour la préparation d'une composition pharmaceutique pour la thérapie ou la prophylaxie des infections virales.

4.- Utilisation d'une composition selon la revendication 2 pour la préparation d'une composition pharmaceutique pour la thérapie ou la prophylaxie des anomalies immunorégulatrices.

5.- Utilisation d'une composition selon la revendication 2 pour la préparation d'une composition pharmaceutique pour la thérapie ou la prophylaxie des néoplasmes.

6.- Utilisation selon l'une des revendications 1 à 5, caractérisée en ce que le système tampon phosphaté contient du phosphate disodique et du phosphate monosodique.

7.- Utilisation selon l'une des revendications 2 à 5, caractérisée en ce que la composition contient de 25 à 100 g/l de polyphosphate.

8.- Utilisation selon la revendication 7, caractérisée en ce que la composition contient 50 g/l de polyphosphate.

9.- Utilisation selon la revendication 7, caractérisée en ce que la composition contient 25 à 100 g/l de solution de polyphosphate à pH 7,5.

10.- Utilisation selon la revendication 8, caractérisée en ce que la composition contient 50 g/l de solution de polyphosphate à pH 7,5.

11.- Utilisation selon la revendication 9 ou 10, caractérisée en ce que la composition contient du polyphosphate de potassium.

12.- Utilisation selon l'une des revendications 1 à 11, caractérisée en ce que l'interféron est un interféron humain.

13.- Utilisation selon la revendication 12, caractérisée en ce que l'interféron humain est un IFN-γ naturel ou recombinant.

14.- Utilisation selon la revendication 13, caractérisée en ce que l'interféron contient du rIFN-γ D0.

15.- Utilisation selon la revendication 13, caractérisée en ce que l'interféron contient du rIFN-γ D3.

16.- Utilisation d'une composition contenant de l'IFN-γ D3 humain recombinant, 1.000 mmol/l de tampon au phosphate de sodium à pH 7,5 et 50 g/l de polyphosphate de potassium selon l'une des revendications 2 à 5.

17.- Procédé de préparation d'une composition contenant de l'interféron, un système tampon phosphaté et/ou du polyphosphate, caractérisé en ce que l'on ajoute du phosphate et/ou du polyphosphate à une solution d'interféron.

ABB. 1

x 1000 E/ml rIFN-gamma

Anfangsanalyse

nach einem Tag Inkubation

nach zwei Tagen Inkubation

pH-Wert

EP 0 219 073 B1

Abb. 2

ABB. 3

x 1000 E/ml rIFN-gamma

Natriumphosphat-Konzentration (mmol/l)

EP 0 219 073 B1

ABB. 4

Abb. 5

ABB. 6